# EUROPÄISCHE PATENTSCHRIFT

(11) **EP 0 558 454 B1**
(45) Veröffentlichungstag und Bekanntmachung des Hinweises auf die Patenterteilung: **22.01.1997**
(21) Anmeldenummer: 93810114.4
(22) Anmeldetag: 22.02.1993
(51) Int. Cl.: C07C 67/29, C07C 69/30, C07C 41/09

(54) **Kondensate und deren Verwendung als Oxalkylierungshilfsmittel**
Condensates and their use as oxyalkylation adjuvants
Condensats et leur utilisation comme adjuvants d'oxyalkylation

(30) Priorität: 26.02.1992 DE 4205844
(43) Veröffentlichungstag der Anmeldung: 01.09.1993
(73) Patentinhaber: Clariant Finance (BVI) Limited, Road Town, Tortola (VG)
(72) Erfinder: Baumann, Hans-Peter, CH-4107 Ettingen (CH)
(74) Vertreter: D'haemer, Jan Constant

(56) Entgegenhaltungen:
- EP-A- 0 335 295
- EP-A- 0 353 928
- DE-B- 1 270 542

## Beschreibung

Oxalkylierte, insbesondere oxäthylierte und/oder oxypropylierte Produkte werden heutzutage als Tenside in vielen Gebieten der Technik eingesetzt, z.B. bei der Resterdölgewinnung, in der Kunststoffindustrie, in Kosmetika, bei der Lebensmittelverarbeitung, bei der Behandlung von textilen oder nicht-textilen Substraten, bei der Herstellung von Farben und Lacken, bei der Verarbeitung von Erzen oder Erden oder von Abwässern oder Schlämmen, sowie bei manchen Rezyklisierungsverfahren usw., wobei eine besonders erwähnenswerte Gruppe von Tensiden durch die oxalkylierten Fettsäureester, besonders Triglyceride, dargestellt ist. Es ist daher gewünscht, daß die Herstellung von solchen Tensiden auf möglichst wirtschaftliche und ausgiebige Weise durchgeführt werden könne und daß dabei in den Endprodukten die Menge an nicht-umgesetzten Alkylenoxyden und/oder an unerwünschten Nebenprodukten (bei Oxäthylierungen insbesondere 1,4-Dioxan) so niedrig wie möglich sei.

Aus dem Beispiel 1 der EP 353 928 A2 ist ein Kondensationsprodukt von Kaliumhydroxyd mit Glycerin bekannt, welches einen K-Gehalt von 4,73 % aufweist und als Substrat zur Propoxylierung dient; das propoxylierte und gegebenenfalls von Kalium befreite Produkt dient zur Umesterung von definierten Fettsäurealkylestern, vorzugsweise in Gegenwart von Kalium- oder Natriummethylat als Katalysator, wenn das propoxylierte Produkt von Kalium befreit wurde. Aus der DE-AS 1 270 542 sind bestimmte Alkalimetallalkoholate aus bestimmten einfachen Alkanolen oder bestimmten Diolen bekannt, die als Katalysatoren für die Anlagerung von Alkylenoxyd an Triglyceride dienen.

Es wurde nun gefunden, daß bestimmte KOH/Oligohydroxyalkankondensate, wie sie unten definiert sind, sich überraschend gut als Katalysatoren für Oxalkylierungen, insbesondere auch für schwierigste Oxalkylierungen von Fettsäureestern, eignen.

Die Erfindung betrifft die Kondensate, deren Herstellung und deren Einsatz als Hilfsmittel, insbesondere als Katalysatoren, für Oxalkylierungen, sowie die damit erhaltenen Oxalkylierungsprodukte.

Ein erster Gegenstand der Erfindung ist also.ein Verfahren zur Herstellung eines Kondensates (K), das dadurch gekennzeichnet ist, daß man
(A) mindestens ein Oligohydroxyalkan, das 3 bis 8 Kohlenstoffatome enthält und mindestens 3 Hydroxygruppen enthält,
unter Zugabe von
(B) Kaliumhydroxyd
im Verhältnis von 2 bis 26 Val (A) pro Mol (B) kondensiert, so daß das Kondensationsprodukt (K) mindestens 5 Gew.-% K⁺ und mindestens eine alkoholische Hydroxygruppe enthält.

Die Oligohydroxyverbindungen (A) enthalten nicht mehr alkoholische Hydroxygruppen als C-Atome und sind im allgemeinen bekannte Verbindungen. Als Oligohydroxyalkane (A) kommen vornehmlich C₄₋₈-Alkantriole und Verbindungen der Formel 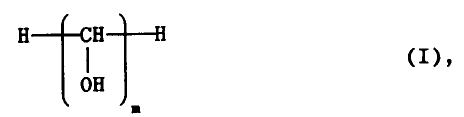 worin m 3 bis 6 bedeutet, in Betracht.

In den Oligohydroxyalkanen (A) können die Alkanketten linear sein oder, wenn sie 4 oder mehr Kohlenstoffatome enthalten, auch verzweigt sein oder, wenn sie 5 oder mehr Kohlenstoffatome enthalten (insbesondere sechs Kohlenstoffatome), können sie auch zyklisch sein. Es seien insbesondere genannt 1,2,4-Butantriol, 1,2,3- oder 1,2,6-Hexantriol, 1,2,3-Heptantriol oder 1,2,3-octantriol und, als Verbindungen der Formel (I), insbesondere Glycerin, Erythrit, Arabit, Xylit, Mannit oder Sorbit. Unter den genannten Verbindungen sind diejenigen mit 3 bis 6 Kohlenstoffatomen bevorzugt, insbesondere diejenigen der Formel (I), vor allem Glycerin. Die Oligohydroxyalkane (A) können als reine Substanzen (z.B. > 95 %-ig) oder auch in technischer Qualität (z.B. 85-95 %-ig) eingesetzt werden.

Das Kaliumhydroxyd (B) kann als reine Substanz oder auch als technisches Kaliumhydroxyd eingesetzt werden, z.B. als 80 bis 100 %-iges KOH, wobei 85 bis 90 %-iges technisches KOH für das erfindungsgemäße Verfahren gut einsetzbar ist.

Die relativen Mengen an (A) und (B) werden zweckmäßig so gewählt, daß im Endprodukt mindestens 5 Gew.-% K⁺ vorhanden sind und mindestens eine Hydroxygruppe durchschnittlich pro Molekül Produkt im Endprodukt enthalten ist. Pro Mol (B) werden 2 bis 26 Val (A) eingesetzt [1 Val (A) = 1 Mol (A) geteilt durch die Anzahl alkoholische Hydroxygruppen im Molekül]. Besonders vorteilhaft werden pro Mol (B) 1 bis 8 Mol (A) eingesetzt; vorteilhaft 6 bis 20 Val, insbesondere 2 bis 8 Mol, vorzugsweise 3 bis 7,5 Mol (A) pro Mol (B).

Die Kondensation von (A) in Gegenwart von (B) erfolgt zweckmäßig unter inerter Atmosphäre, vorzugsweise unter Stickstoff, und unter Reaktionswasserentzug in der Wärme, vorteilhaft bei Temperaturen > 60°C; obwohl es auch möglich ist, bei Temperaturen bis z.B. 160°C zu kondensieren, beispielsweise zwischen 100 und 120°C, ist es bevorzugt, die Kondensation bei Temperaturen im Bereich von 60 bis 100°C, vorzugsweise 75 bis 98°C, durchzuführen und das Reaktionswasser unter vermindertem Druck zu entziehen. Die Kondensation wird vorteilhaft so lange weitergeführt, bis die Bildung von Reaktionswasser abklingt und das ganze vorhandene Kalium des eingesetzten Kaliumhydroxyds den Wasserstoff von alkoholischen Hydroxygruppen ersetzt hat und vorzugsweise ein Teil der übrigen Hydroxygruppen zu Äthergruppen kondensiert ist, so daß im Reaktionsprodukt auch Oligokondensate von (A) als Kaliumsalze vorhanden sind. Vorteilhaft wird die Kondensation so lange weitergeführt, bis 2 bis 200, vorzugsweise 5 bis 50 Mol-% überschüssiges Kondensationswasser über das aus der einfachen Kaliumalkoholatbildung entstehende entzogen wird. Vorzugsweise wird das Kondensationswasser erst nach Abklingen seiner Bildung entzogen (vorzugsweise unter vermindertem Druck). Die gesamte Kondensation, einschließlich Wasserentzug, wird vorteilhaft innerhalb von 2 bis 10, vorzugsweise 2½ bis 6 Stunden durchgeführt, wobei die Kondensationsreaktion z.B. 1,5 bis 3 Stunden dauern kann und die restliche Zeit im wesentlichen durch die verwendete Apparatur und die Vakuumsteuerung für den Kondensationswasserentzug bedingt ist. Je nach eingesetzter Verbindung (A) und Gewichtsverhältnis (A)/(B) können die geeigneten Kondensationstemperaturen und -dauern variieren und durch einige Vorversuche optimiert werden. Vorteilhaft werden die relativen Mengen an (A) und (B) so gewählt, daß im erhaltenen Kondensat (K) der K⁺-Gehalt im Bereich von 5 bis 22, vorzugsweise 5,4 bis 22, insbesondere 7 bis 15 Gew.-% liegt.

Die so hergestellten Kondensate (K) sind im allgemeinen flüssig bis dickflüssig oder auch pastös bis fest, und praktisch geruchlos; sie sind gut wasserlöslich, sowie auch gut löslich in oder mischbar mit vegetabilen Ölen. Die flüssigen bis dickflüssigen Produkte sind bevorzugt. Vorzugsweise beträgt deren Rotationsviskosität bei 60°C 300 bis 2000 mPa·s.

Die nach obigem Verfahren erhältlichen Kondensate (K) dienen als Hilfsmittel, insbesondere als Katalysatoren, für Oxalkylierungen, insbesondere mit
(C) mindestens einem cyclischen D-Oxyd,
worin D Äthylen, Propylen, Butylen oder Styrol bedeutet.

Als Oxalkylierungen kommen hier sowohl solche von Verbindungen, die aktive Wasserstoffe enthalten (vornehmlich von Alkoholen, Aminen, Carbonsäuren oder Carbonsäureamiden), als auch solche von Verbindungen, die keine solchen aktiven Wasserstoffatome enthalten (insbesondere von gesättigten und/oder ungesättigten Fettsäureestern), in Betracht. Besonders hervorzuheben sind die Oxalkylierungen von
(F) natürlichen, gegebenenfalls modifizierten, estergruppenhaltigen Ölen, Fetten, Wachsen und/oder Harzen.

Als modifizierte Öle, Fette, Wachse oder harze kommen vor allem estergruppenhaltige Modifikationen in Betracht (z.B. deren Teilverseifungs-, Hydrierungs-, Oxydations-, Alkoholyse- und/oder Umesterungsprodukte und/oder Oligomerisationsprodukte von ungesättigten Fettsäureestern). Besonders hervorzuheben ist die Oxalkylierung von Fettsäuretriglyceriden, wie sie als natürliche Öle oder Fette vorkommen, sei es als technische Triglyceridgemische oder als raffinierte Triglyceride, worin die esterbildenden Fettsäuren beispielsweise 6 bis 24 Kohlenstoffatome enthalten und gesättigt, ungesättigt und/oder hydroxygruppenhaltig sein können, oder noch von deren Alkoholyse- bzw. Umesterungsprodukten zu Fettsäureestern niederer Alkanole (z.B. Methylestern oder Äthylestern) oder von natürlichen Estern höherer Alkohole. Es seien beispielsweise die folgenden erwähnt: pflanzliche und tierische Öle oder Fette (z.B. Baumwollsaatöl, Erdnußöle, Leinöl, Maisöl, Olivenöle, Rapsöle, Saffloröl, Sesamöl, Sojaöl, Sonnenblumenöl, Palmkernöl, Palmöl, Kokosöl, Kakaobutter, Sheafett, Butterfett, Rindertalg, Hammeltalg, Schweineschmalz, Anschovisöl, Walöl, Robbenöl, Makrelenöl, Heringsöl, Kapelanöl, Jojobaöl, Ricinusöl und Babassuöl), hydrierte Öle, Metiloil, pflanzliche oder tierische rezente Wachse (Carnaubawachs, Japanwachs, Candelillawachs, Reiskeimölwachs, Ouricurywachs, Bienenwachs, Schellackwachs, Lanolin), sowie fossile, pflanzliche Wachse (z.B. Montanwachs), estergruppenhaltige Mineralwachse (Ceresin, Ozokerit) und estergruppenhaltige oxydierte Kohlenwasserstoffwachse, oder noch teilverseifte Triglyceride. Besonders hervorzuheben darunter sind die natürlichen vegetabilen Öle, vor allem diejenigen, worin mindestens 35 Mol-%, vorzugsweise mindestens 50 Mol-% der esterbildenden Fettsäuren ungesättigte Säuren sind (vornehmlich Myristolsäure, Palmitolsäure, Ölsäure, Gadoleinsäure, Erucasäure, Linolsäure und/oder Linolensäure), die Talgfette (vornehmlich Rindertalg, Hammeltalg, Schweineschmalz und gemischte Talgfette) und die Methylester der jeweiligen Fettsäuregemische (z.B. Metiloil).

Ferner können z.B. Sorbitan-mono-, -di- oder -triester von höheren Fettsäuren eingesetzt werden, z.B. Sorbitanmono-, -di- oder -tri-laurat, -palmitat, -oleat oder -stearat.

Zur Oxalkylierung können als (C) übliche Alkylenoxyde (insbesondere Oxirane) verwendet werden, z.B. Äthylenoxyd, Propylenoxyd, Butylenoxyd und/oder Styroloxyd. Da die Oxalkylierung, insbesondere von (F), vornehmlich zur Erhöhung der Hydrophilie der Produkte dient, wird dafür vornehmlich Äthylenoxyd eingesetzt und, zusätzlich zum Äthylenoxyd, gegebenenfalls Propylenoxyd, Butylenoxyd und/oder Styroloxyd. Vorteilhaft ist mindestens die Hälfte (d.h. mindestens 50 Mol-%) der eingesetzten Oxirane (C) Äthylenoxyd; besonders vorteilhaft sind mindestens 80 Mol-% davon Äthylenoxyd. Vorzugsweise werden mindestens 10 Mol, vorteilhaft 10 bis 200 Mol, insbesondere 20 bis 150 Mol Äthylenoxyd pro Mol Substrat, besonders (F), angelagert; andere Oxyde (C) werden, wenn sie auch eingesetzt werden, vorzugsweise in solchen Mengen eingesetzt, die 1 bis 20 Mol-%, bevorzugt 1 bis 10 Mol-% der eingesetzten Menge Äthylenoxyd entsprechen.

Besonders hervorzuheben ist die Verwendung von Kondensationsprodukten (K), worin (A) Glycerin ist, vor allem bei der Oxalkylierung von Triglyceriden.

Die Oxalkylierung kann analog zu üblichen Oxalkylierungen durchgeführt werden, im allgemeinen unter inerter Atmosphäre (z.B. durch Verdrängung von Luftsauerstoff mit Stickstoffgas), unter normalem, vermindertem oder auch erhöhtem Druck und bei Temperaturen, die vorteilhaft im Bereich von 110 bis 200°C, vorzugsweise ≤ 180°C, insbesondere im Bereich von 140 bis 180°C liegen. Die meisten Öle lassen sich erfindungsgemäß bei Temperaturen im Bereich zwischen 150 und 180°C, insbesondere bei 160 bis 175°C, hervorragend oxalkylieren.

Das erfindungsgemäße Oxalkylierungshilfsmittel (K) wird zweckmäßig in solchen Mengen eingesetzt, daß die Oxalkylierung vonstatten gehen kann; das erfindungsgemäße Hilfsmittel (K) wird vorteilhaft in solchen Mengen eingesetzt, die mindestens 2 g, vorzugsweise 2 bis 25 g, insbesondere 3 bis 15 g K⁺ pro Mol Substrat, insbesondere pro Mol (F), entsprechen. Die geeignete und insbesondere die optimale Menge (K) kann je nach Substrat und Alkylenoxyd variieren und kann durch einige Vorversuche ermittelt bzw. optimiert werden.

Als orientative Prüfmethode kann z.B. als Maßstab gesetzt werden, daß eine bestimmte Art und Menge Katalysator (K) für die Oxäthyllerung eines bestimmten Substrates, insbesondere (F), dann gut geeignet ist, wenn bei 170°C und bei Normaldruck, nach Verdrängung des Luftsauerstoffes mit Stickstoffgas und Einleitung von Äthylenoxyd, die Oxäthylierung innerhalb von drei Stunden eingesetzt hat.

Zweckmäßig werden das Substrat, insbesondere (F), und der Katalysator (K) so gewählt, daß das Gemisch mindestens bei der Oxalkylierungstemperatur flüssig ist und (K) in dem zu oxalkylierenden Substrat, insbesondere in (F), gelöst ist.

Durch das erfindungsgemäße Verfahren und unter Verwendung des erfindungsgemäßen Katalysators (K) können Oxalkylierungen auch von sonst sehr schwierig zu oxalkylierenden Substraten, insbesondere von Ölen oder Fetten, die frei von aktiven Wasserstoffen sind, überraschend leicht und gut durchgeführt werden, wobei die Ausbeute an oxalkyliertem Produkt sehr hoch ist und die erhaltenen Produkte im wesentlichen frei von unerwünschten Nebenprodukten sind. Durch die erfindungsgemäße Oxalkylierung von Fettsäureestern (F) sind oxalkylierte Produkte erhältlich, deren Hydroxylzahl je nach Substrat, Katalysator (K) und Bedingungen variieren kann und im allgemeinen relativ hoch ist (sie liegt vornehmlich im Bereich von 30 bis 300), aber jeweils niedriger ist als die Hydroxylzahl die vergleichsweise durch die entsprechende Oxalkylierung der jeweiligen (F)-Hydrolysate erhältlich ist, und zwar beträgt sie vornehmlich 2 bis 98, vorzugsweise 10 bis 95, insbesondere 50 bis 95 % davon.

Die erfindungsgemäßen hochwirksamen Katalysatoren (K) sind auf verblüffend einfache Weise und in optimaler Ausbeute wie hier beschrieben herstellbar und zeichnen sich durch ihre Wirksamkeit und Lagerbeständigkeit aus.

In den folgenden Beispielen bedeuten die Prozente Gewichtsprozente; die Temperaturen sind in Celsiusgraden angegeben. Die in den Beispielen A bis T hergestellten Oxäthylierungsprodukte sind umweltfreundlich und biologisch abbaubar, sie haben tensiden Charakter und sind als Emulgatoren und als Egalisiermittel für Dispersionsfarbstoffe geeignet.

### Beispiel 1

In einem 5-Hals-Sulfierkolben werden 460,5 g Glycerin mit 62,3 g technischem 90 %-igem KOH unter N₂ und guter Rührung auf 90°C Innentemperatur aufgeheizt. Man rührt das Gemisch zwei Stunden bei dieser Temperatur, destilliert sodann bei 135 mbar das Reaktionswasser ab und verstärkt nach und nach das Vakuum (nach 30 Minuten auf 100 mbar und nach 60 Minuten auf 35 mbar) und destilliert bei 30-35 mbar weiter bis kein Wasser mehr übergeht (ca. ein bis zwei Stunden). Dann stellt man die Heizung ab, läßt abkühlen und läd aus. Es resultieren 494,2 g Katalysator (K₁); es werden 23,6 g klares Reaktionswasser als Destillat aufgefangen, wobei ein kleiner Teil (ca. 3 g) durch das Vakuum mitgerissen wird.

Das Produkt (K₁) enthält 7,9 % K⁺.
Viskosität bei 60°C = 750 mPa·s.

Die folgende Tabelle enthält weitere Beispiele von erfindungsgemäßen Katalysatoren (K₂) bis (K₉), die analog wie im Beispiel 1 beschrieben hergestellt werden und durch die Komponente (A) und den K⁺-Gehalt gekennzeichnet sind.

### Beispiele 2 bis 9 [Katalysatoren (K₂) bis (K₉)]

| (K) | (A) | % K⁺ |
|---|---|---|
| (K₂) | Glycerin | 13,96 |
| (K₃) | Glycerin | 21,56 |
| (K₄) | Glycerin | 16,2 |
| (K₅) | Glycerin | 8,86 |
| (K₆) | Glycerin | 9,0 |
| (K₇) | Butantriol-1,2,4 | 8,5 |
| (K₈) | 1,2,6-Hexantriol | 5,49 |
| (K₉) | D-Sorbit | 8,84 |

### OXALKYLIERUNGSBEISPIELE

### Beispiel A

110,0 g technisches Rüböl (mittleres Molekulargewicht MG = 880) und 5,5 g Katalysator (K6) werden in einen 5-Hals-Sulfierkolben von 500 ml Fassungsvermögen gegeben und unter Rühren auf 115-125°C aufgeheizt, dann wird drei Mal hintereinander jeweils mit Wasserstrahlvakuum (ca. 40 mbar) evakuiert und mit Stickstoff entlastet; nun wird ein viertes Mal evakuiert und dann mit Äthylenoxyd entlastet. Innerhalb von vier Stunden werden bei 160-170°C 275,0 g Äthylenoxyd angelagert.

Man erhält 390,5 g Oxäthylierungsprodukt (Additionsprodukt von 50 Mol Äthylenoxyd an 1 Mol Rüböl in Gegenwart des eingesetzten Katalysators).

### Beispiel B

Man verfährt wie im Beispiel A beschrieben, mit dem Unterschied, daß anstelle von 5,5 g Katalysator (K₆) 3,3 g Katalysator (K₂) eingesetzt werden und pro Mol Rüböl 30 Mol Äthylenoxyd angelagert werden.

### Beispiel C

Man verfährt wie im Beispiel A beschrieben, mit dem Unterschied, daß anstelle von 5,5 g Katalysator (K₆) 1,9 g Katalysator (K₆) eingesetzt werden und 120 Mol Mol Äthylenoxyd pro Mol Rüböl angelagert werden.

### Beispiel D

Man verfährt wie im Beispiel A beschrieben, mit dem Unterschied, daß anstelle von 5,5 g des Katalysators (K₆) 11 g des Katalysators (K₇) eingesetzt werden und pro Mol Rüböl 10,1 Mol Äthylenoxyd angelagert werden.

### Beispiel E

Man verfährt wie im Beispiel D beschrieben, mit dem Unterschied, daß anstelle von 11 g des Katalysators (K₇) 11 g des Katalysators (K₈) eingesetzt werden und pro Mol Rüböl 20 Mol Äthylenoxyd angelagert werden.

### Beispiel F

Man verfährt wie im Beisplel A beschrieben, mit dem Unterschied, daß anstelle von Rüböl Sojaöl eingesetzt wird und pro Mol Sojaöl 40 Mol Äthylenoxyd angelagert werden.

### Beispiel G

Man verfährt wie im Beispiel F beschrieben, mit dem Unterschied, daß pro Mol Sojaöl 20 Mol Äthylenoxyd angelagert werden.

### Beispiel H

Man verfährt wie im Beispiel A beschrieben, mit dem Unterschied, daß pro Mol Rüböl 70 Mol Äthylenoxyd angelagert werden.

### Beispiel I

Man verfährt wie im Beispiel A beschrieben, mit dem Unterschied, daß anstelle von 5,5 g des Katalysators (K₆) 2,75 g des Katalysators (K₃) eingesetzt werden und pro Mol Rüböl 20 Mol Äthylenoxyd angelagert werden.

### Beispiel J

Man verfährt wie im Beispiel A beschrieben, mit dem Unterschied, daß anstelle der 5,5 g des Katalysators (K₆) 11 g des Katalysators (K₁) eingesetzt werden und 24 Mol Äthylenoxyd pro Mol Rüböl angelagert werden.

### Beispiel K

Man verfährt wie im Beispiel A beschrieben, mit dem Unterschied, daß anstelle von Rüböl die gleiche Menge an Metiloil eingesetzt wird und anstelle der 5,5 g des Katalysators (K₆) 22 g des Katalysators (K₅) eingesetzt werden und 35 Mol Äthylenoxyd pro Mol Metiloil angelagert werden.

### Beispiel L

Man verfährt wie im Beispiel K beschrieben, mit dem Unterschied, daß anstelle von 22 g des Katalysators (K₅) 16,5 g davon verwendet werden und 14,2 Mol Äthylenoxyd pro Mol Metiloil angelagert werden.

(Metiloil ist ein käufliches technisches Fettsäuremethylestergemisch mit Verseifungszahl 170-200, Säurezahl 1-12 und durchschnittlichem Molekulargewicht MG = 298).

### Beispiel M

Man verfährt wie im Beispiel A beschrieben, mit dem Unterschied, daß anstelle der 5,5 g des Katalysators (K₆) 11 g des Katalysators (K₉) eingesetzt werden und 40 Mol Äthylenoxyd pro Mol Rüböl angelagert werden.

### Beispiel N

Man verfährt wie im Beispiel A beschrieben, mit dem Unterschied, daß anstelle von Rüböl Sojaöl eingesetzt wird und anstelle der 5,5 g des Katalysators (K₆) 11 g des Katalysators (K₉) eingesetzt werden und pro Mol Sojaöl 50 Mol Äthylenoxyd angelagert werden.

### Beispiel O

Man verfährt wie im Beispiel N beschrieben, mit dem Unterschied, daß anstelle von Sojaöl Sonnenblumenöl eingesetzt wird und 35 Mol Äthylenoxyd pro Mol Sonnenblumenöl angelagert werden.

### Beispiel P

Man verfährt wie im Beispiel O beschrieben, mit dem Unterschied, daß anstelle der 11 g Katalysator (K₉) 11 g Katalysator (K₆) eingesetzt werden und pro Mol Sonnenblumenöl 50 Mol Äthylenoxyd angelagert werden.

### Beispiel Q

Man verfährt wie im Beispiel A beschrieben, mit dem Unterschied, daß anstelle von Rüböl Jojobaöl eingesetzt wird und anstelle der 5,5 g des Katalysators (K₆) 11 g des Katalysators (K₆) eingesetzt werden und pro Mol Jojobaöl 30 Mol Äthylenoxyd angelagert werden.

### Beispiel R

Man verfährt wie im Beispiel Q beschrieben, mit dem Unterschied, daß anstelle von Jojobaöl die gleiche Menge Schweineschmalz eingesetzt wird.

### Beispiel S

Man verfährt wie im Beispiel Q beschrieben, mit dem Unterschied, daß anstelle von Jojobaöl die gleiche Menge Ricinusöl eingesetzt wird.

### Beispiel T

Man verfährt wie im Beispiel N beschrieben, mit dem Unterschied, daß anstelle von Sojaöl Sorbitantrioleat eingesetzt wird und 12 Mol Äthylenoxyd pro Mol Sorbitantrioleat angelagert werden.

## Patentansprüche

1. Verfahren zu Herstellung eines Kondensates (K), dadurch gekennzeichnet, daß man
(A) mindestens ein Oligohydroxyalkan, das 3 bis 8 Kohlenstoffatome enthält und mindestens 3 Hydroxygruppen enthält,
unter Zugabe von
(B) Kaliumhydroxyd
im Verhältnis von 2 bis 26 Val (A) pro Mol (B) kondensiert, so daß das Kondensationsprodukt (K) mindestens 5 Gew.-% K⁺ und mindestens eine alkoholische Hydroxygruppe enthält.

2. Verfahren nach Anspruch 1, dadurch gekennzeichnet, daß man als (A) ein C₄₋₈-Alkantriol oder eine Verbindung der Formel 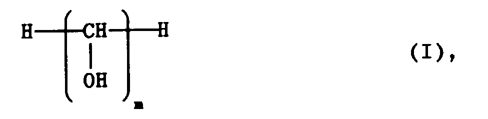 worin m 3 bis 6 bedeutet,
oder ein Gemisch solcher Verbindungen einsetzt.

3. Verfahren gemäß Anspruch 1 oder 2, dadurch gekennzeichnet, daß man unter inerter Atmosphäre und unter Reaktionswasserentzug, bei 60 bis 100°C kondensiert.

4. Die nach dem Verfahren gemäß einem der Ansprüche 1 bis 3 erhältlichen Kondensate (K).

5. Kondensate (K) gemäß Anspruch 4 mit einem K⁺-Gehalt von 5 bis 22 Gew.-%.

6. Verwendung der Kondensate (K) gemäß Anspruch 4 als Hilfsmittel bei Oxalkylierungen.

7. Verwendung gemäß Anspruch 6 bei der Oxalkylierung von
(F) natürlichen, gegebenenfalls modifizierten, estergruppenhaltigen Ölen, Fetten, Wachsen und/oder Harzen.

8. Verwendung gemäß Anspruch 6 oder 7, dadurch gekennzeichnet, daß man zur Oxalkylierung
(C) mindestens ein cyclisches D-Oxyd,
worin D Äthylen, Propylen, Butylen oder Styren bedeutet,
einsetzt.

9. Verwendung gemäß Anspruch 8, dadurch gekennzeichnet, daß man als (C) Äthylenoxyd und gegebenenfalls Propylenoxyd, Butylenoxyd und/oder Styroloxyd einsetzt.

10. Verfahren zur Oxalkylierung von mindestens einem natürlichen, gegebenenfalls modifizierten, estergruppenhaltigen Öl, Fett, Wachs oder Harz (F), das wie im Anspruch 7 definiert ist, dadurch gekennzeichnet, daß man (F) mit (C), das wie in Anspruch 8 oder 9 definiert ist, in Gegenwart von (K), das wie im Anspruch 4 oder 5 definiert ist, umsetzt.

11. Verfahren gemäß Anspruch 10, dadurch gekennzeichnet, daß man bei Temperaturen ≤ 180°C, vorzugsweise im Bereich von 150 bis 180°C, oxalkyliert.

12. Verfahren gemäß Anspruch 10 oder 11, dadurch gekennzeichnet, daß man (K) in solchen Mengen einsetzt, die 2 bis 25 g K⁺ pro Mol (F) entsprechen.

## Claims

1. Process for the production of a condensate (K), characterized in that
(A) at least one oligohydroxyalkane, which contains 3 to 8 carbon atoms and at least 3 hydroxy groups
is condensed with the addition of
(B) potassium hydroxide
at the ratio of 2 to 26 vals of (A) per mole of (B), so that the condensation product (K) contains at least 5 % by weight of K⁺ and at least one alcoholic hydroxy group.

2. Process according to Claim 1, characterized in that as (A) there is employed a C₄₋₈-alkanetriol or a compound of formula 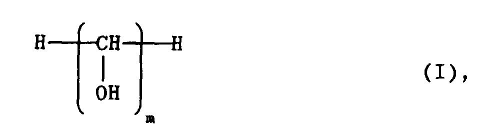 wherein m signifies 3 to 6,
or a mixture of such compounds.

3. Process according to Claim 1 or 2, characterized in that the condensation is carried out at 60 to 100°C under an inert atmosphere and with elimination of the reaction water.

4. The condensates (K) obtainable by the process according to any one of Claims 1 to 3.

5. Condensates (K) according to Claim 4 with a K⁺-content of 5 to 22 % by weight.

6. Use of the condensates (K) according to Claim 4 as an adjuvant in oxyalkylations.

7. Use according to Claim 6 in the oxyalkylation of
(F) natural, optionally modified ester group-containing oils, fats, waxes and/or resins.

8. Use according to Claim 6 or 7, characterized in that for oxyalkylation there is employed
(C) at least one cyclic D-oxide,
wherein D signifies ethylene, propylene, butylene or styrene.

9. Use according to Claim 8, characterized in that as (C) there is employed ethylene oxide and optionally propylene oxide, butylene oxide and/or styrene oxide.

10. Process for the oxyalkylation of at least one natural, optionally modified, ester group-containing oil, fat, wax or resin (F), which is defined as in Claim 7, characterized in that (F) is reacted with (C), which is defined as in Claim 8 or 9, in the presence of (K), which is defined as in Claim 4 or 5.

11. Process according to Claim 10, characterized in that the oxyalkylation is carried out at temperatures ≤ 180°C, preferably in the range of 150 to 180°C.

12. Process according to Claim 10 or 11, characterized in that (K) is employed in such an amount that corresponds to 2 to 25 g of K⁺ per mol of (F).

## Revendications

1. Un procédé de préparation d'un condensat (K), caractérisé en ce que l'on condense
(A) au moins un oligohydroxyalcane qui contient de 3 à 8 atomes de carbone et au moins 3 groupes hydroxy,
avec addition de
(B) hydroxyde de potassium
dans une proportion de 2 à 26 Val de (A) par mole de (B), de sorte que le produit de condensation (K) contient au moins 5% en poids de K⁺ et au moins un groupe hydroxy alcoolique.

2. Un procédé selon la revendication I, caractérisé en ce qu'on utilise comme (A) un C_{4,8}-alcanetriol ou un composé de formule 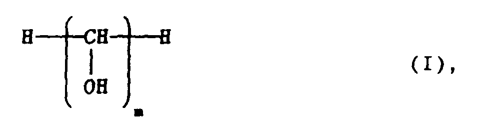 où m signifie de 3 à 6,
ou un mélange de tels composés.

3. Un procédé selon la revendication 1 ou 2, caractérisé en ce qu'on effectue la condensation à une température comprise entre 60 et 100°C, sous atmosphère inerte et avec élimination de l'eau de réaction.

4. Un condensat (K) pouvant être obtenu selon le procédé de l'une des revendications 1 à 3.

5. Un condensat (K) selon la revendication 4 avec une teneur en K⁺ comprise entre 5 et 22% en poids.

6. Utilisation d'un condensat (K) selon la revendication 4 comme auxiliaire pour des oxyalkylations.

7. L'utilisation selon la revendication 6 pour oxyalkyler
(F) des huiles, des graisses, des cires et/ou des résines naturelles contenant des groupes esters, éventuellement modifiées.

8. L'utilisation selon la revendication 6 ou 7, caractérisée en ce que pour l'oxyalkylation, on utilise
(C) au moins un D-oxyde cyclique,
où D signifie un groupe éthylène, propylène, butylène ou styrène.

9. L'utilisation selon la revendication 8, caractérisée en ce qu'on utilise comme (C) de l'oxyde d'éthylène et éventuellement de l'oxyde de propylène, de l'oxyde de butylène et/ou de l'oxyde de styrène.

10. Un procédé pour l'oxyalkylation de (F) qui est au moins une huile, une graisse, une cire ou une résine naturelles contenant des groupes esters, éventuellement modifiées telles que définies à la revendication 7, caractérisé en ce qu'on fait réagir (F) avec (C) tel que défini à la revendication 8 ou 9, en présence de (K) tel que défini à la revendication 4 ou 5.

11. Un procédé selon la revendication 10, caractérisé en ce qu'on effectue l'oxyalkylation à des températures ≤ 180°C, de préférence comprises entre 150 et 180°C.

12. Un procédé selon la revendication 10 ou 11, caractérisé en ce qu'on utilise (K) en une quantité telle qu'elle corresponde de 2 à 25 g de K⁺ par mole de (F).
